# EUROPÄISCHE PATENTANMELDUNG

(11) **EP 1 000 630 A1**
(43) Veröffentlichungstag der Anmeldung: **17.05.2000**
(21) Anmeldenummer: 99121825.6
(22) Anmeldetag: 04.11.1999
(51) Int. Cl.: A61M 1/00

(54) **Verfahren zur Bildung einer Infektionsschutzmanschette und Vorrichtung zur Durchführung des Verfahrens**

(30) Priorität: 11.11.1998 DE 19852848
(71) Anmelder: Affeld, Klaus, Prof. Dr. Ing., 14050 Berlin (DE)
(72) Erfinder: Affeld, Klaus Prof. Dr. Ing., 14050 Berlin (DE)
(74) Vertreter: Linser, Heinz, Phys.

(57) **Zusammenfassung**

Die Erfindung betrifft ein Verfahren zur Bildung einer künstlichen Hautdurchleitung für die Durchführung eines Kanals durch die menschliche oder tierische Haut. Dieses Verfahren ist durch die Bildung einer Infektionsschutzmanschette gekennzeichnet, die den eigentlichen Kanal von der Haut und dem darunterliegendem Gewebe isoliert. Diese Infektionsschutzmanschette bildet sich ständig oder in zeitlichen Abständen neu, sie wächst von innen nach aussen und verhindert so ein Eindringen von Infektionskeimen entlang der Infektionsschutzmanschette in tiefere Schichten des Körpers.

## Beschreibung

Die Erfindung betrifft ein Verfahren zur Bildung einer Infektionsschutzmanschette einer künstlichen Hautdurchleitung für die Durchführung eines Kanals durch die Haut ins Körperinnere.

Eine künstliche Hautdurchleitung hat die Aufgabe, eine Verbindung in der Form eines Kanals zwischen dem Inneren des Körpers und der Aussenwelt herzustellen. Dies ist wichtig für Patienten, bei denen eine Therapie oder Diagnose eine solche Verbindung erfordert. So gibt es eine Anwendung bei der Peritonealdialyse, bei der durch einen in der Bauchwand dauerhaft implantierten Kanal die Dialyseflüssigkeit von aussen in den Körper eingeleitet und auch wieder herausgeleitet wird. Dieser Kanal wird auch als künstliche Hautdurchleitung bezeichnet. Eine weitere Anwendung gibt es bei künstlichen Herzunterstützungssystemen, bei denen durch eine künstliche Hautdurchleitung die Zuleitung von pneumatischer oder elektrischer Energie erfolgt. Eine weitere Anwendung gibt es bei Dauerkathetern, durch die Medikamente oder Nährstofflösungen in den Körper eingeleitet werden. Eine weitere Anwendung gibt es bei elektrischen Leitungen für die Einleitung oder Ausleitung von elektrischen Biosignalen, beispielsweise für die Stimulierung von Muskeln oder für die Messung von Körperfunktionen im Körperinneren.

Bei allen diesen künstlichen Hautdurchleitungen besteht die Gefahr, dass bei längeren Verweilzeiten Infektionen auftreten. Durch sorgfältige Pflege kann man diese zwar in Grenzen beherrschen, jedoch müssen immer wieder Hautdurchleitungen explantiert werden; eine Operation, die auch das Leben des Patienten gefährden kann, wenn die Infektion tief genug in den Körper eingedrungen ist. Die Infektion an einer Hautdurchleitung bildet sich zuerst an der Grenze, an der sich die drei Phasen Implantat, Körpergewebe und keimhaltige Aussenwelt treffen. Dies ist der Bereich, an der die Hautdurchleitung die Haut durchtritt. Die Keime können an dieser Dreiphasenlinie zunächst durch häufige Reinigung und durch äussere Bakteriostatika beherrscht werden. Bei einem längeren Verweilen bilden viele Keime jedoch einen Biofilm auf der Oberfläche der künstlichen Hautdurchleitung. Dieser Biofilm ist eine Schicht von Keimen, die sich am Kanalmaterial anhaftet und gegen die Körperabwehr durch eine Schleimschicht schützt. Da der Körper diesen Biofilm nicht durchdringen kann, kann er die Infektion nicht wirksam bekämpfen. Der Biofilm hat weiterhin die Eigenschaft sich auszudehnen. Er wächst in Richtung der Nährstoffquelle, in diesem Fall in die Richtung des Körperinneren. Dadurch bildet sich eine Tasche, die schwierig zu reinigen ist und die leicht zu einer grösseren Infektion führt.

Es sind zahlreiche künstliche Hautdurchleitungen bekannt, bei denen versucht wird, diese Gefahr durch kragenförmige Ausformungen der Hautdurchleitung zu verhindern. Diese Ausformungen werden unter die Haut implantiert und sollen ein Eindringen des Biofilms verhindern. Die Erfahrung zeigt jedoch, dass auch diese Kragen auf Dauer nicht von der Haut umwachsen bleiben, sondern dass sich die Haut zurückbildet, bis der Kragen freiliegt und infiziert werden kann.

Weiterhin sind künstliche Hautdurchleitungen bekannt, bei denen die Implantatoberfläche mit einem durchwachsbaren Textilgewebe beschichtet ist. Die Erfahrung zeigt jedoch, dass diese durchwachsbaren Gewebe auf Dauer ebenfalls infiziert werden.

In der Patentschrift DE 39 43 412 A1 wird die Katheteroberfläche mit einem durchwachsbaren Textilgewebe beschichtet, das von der Oberfläche aus zum Körpergewebe hin in seiner Porosität abnimmt, um ein leichtes und sicheres Einwachsen des Körpergewebes in das Textilgewebe zu erreichen. Die Erfahrung zeigt jedoch, dass auch diese Katheter auf Dauer nicht vom Körpergewebe umwachsen bleiben, sondern sich auch infizieren.

In der Patentschrift DE 197 28 489 A1 wird die Katheteroberfläche mit einem organischen Gewebe aus Kollagenfasern oder Kollagen-Polymerfasern beschichtet. Die Idee ist, dass das natürliche Kollagen sich günstig auf das Einwachsen und dauerhafte Gedeihen des natürlichen Körpergewebes auswirkt. Die Erfahrung zeigt jedoch, dass auch diese Massnahme Infektionen nicht verhindern kann.

Weiterhin sind künstliche Hautdurchleitungen bekannt, bei denen man durch eine leichtbewegliche Dreiphasenlinie die Einwirkung von mechanischen Kräften auf die anhaftenden Zellen verringern möchte. Dies ist näher dargestellt in einer Arbeit des Anmelders (Grosse-Siestrup, Ch., Affeld, K.: Design criteria for artificial percutaneous devices, Journal of Biomedical Materials Research, Vol 18, 357-382 (1984)). Die Erfahrung zeigt jedoch, dass auch diese Massnahme auf Dauer eine Infektion nicht verhindern kann.

Allen diesen künstlichen Hautdurchleitungen ist gemeinsam, dass sie auf Dauer eine Infektion und ein Eindringen von Keimen in den Körper nicht verhindern können. Sie sind mit Komplikationen verbunden und gefährden den Patienten.
Der Erfindung liegt die Aufgabe zugrunde, die oben erwähnten Nachteile der bisherigen Lösungen zu vermeiden und die Aufgabe auf technisch bessere Weise zu lösen.

Dies wird dadurch erreicht, dass sich eine Infektionsschutzmanschette an der infektionsgefährdeten Dreiphasenlinie beständig oder in Abständen neu bildet und aus dem Körper herausbewegt, wie es im Patentanspruch 1 angegeben ist. In technischer Weise wird so ein Vorgang nachgeahmt, der im Körper auf natürliche Weise erfolgt, so beim Herauswachsen von unbelebten natürlichen Bildungen, z.B. von Haar und Fingernagel. Der sich im Bereich der Dreiphasenlinie bildende Biofilm wird auf diese Weise aus dem Körper herausbewegt und kommt in nährstoffarme und trockene Bereiche, in denen er abstirbt. Die Neubildung der Dreiphasenlinie wird dadurch erreicht, dass sich im keimfreien Körperinneren eine Infektionsschutzmanschette neu bildet. Diese Infektionsschutzmanschette umgibt den eigentlichen funktionstragenden Kanal und trennt ihn vom umgebenden Gewebe. Die Infektionsschutzmanschette wird im Körperinneren aus einem plastischen oder flüssigen Material gebildet, das bei Kontakt mit dem Wasserdampf oder einer anderen Substanz des Körpergewebes aushärtet. Das Material wird vorzugsweise von aussen durch eine Rohrleitung zugeführt. Ein solches Material ist beispielsweise der Silikonkautschuk RTV 3140 der Firma Dow Corning, der bei Raumtemperatur unter dem Einfluss von Wasserdampf - hier bei dieser Anwendung ist es der Wasserdampf des umgebenden Gewebes - aushärtet. Dieser Silikonkautschuk verwandelt sich bei diesem Prozess von einer zähen Flüssigkeit in ein Elastomer. Es sind weitere Materialien bekannt, die unter gleichen Umständen zu gummielastischen Polyurethanen aushärten. Diesen Materialien können ferner Substanzen beigemischt werden, die biologisch wirksam sind und die Wirkung der Hautdurchleitung günstig beeinflussen. So können Antibiotika beigemischt werden, die dann im Körper durch Diffusion ins umgebende Gewebe eindringen und es sicher keimfrei halten. Andere Substanzen können das Anhaften der Zellen an die Infektionsschutzmanschette fördern. Das Anhaften der Zellen kann weiterhin durch eine vergrösserte Oberfläche gefördert werden. Die Infektionsschutzmanschette, die sich in der oben beschriebenen Weise beständig oder in Abständen neu bildet und aus dem Körper herauswächst, wird dann in Abständen - wiederum ähnlich den natürlichen Bildungen wie Haar und Fingernagel - mechanisch abgetrennt und so gekürzt.

Die mit der Erfindung erzielten Vorteile bestehen insbesondere darin, dass Infektionen im ganzen Bereich der Hautdurchleitung vollkommen vermieden werden können.

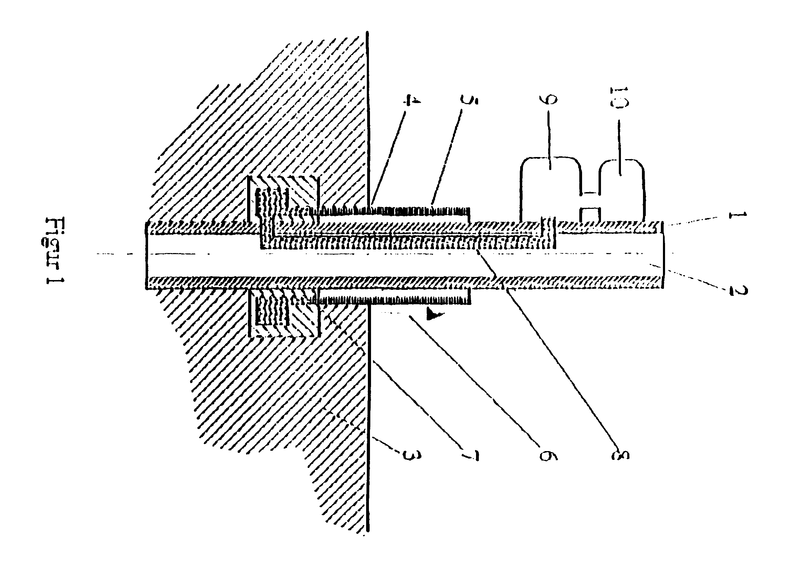

Figur 1 zeigt eine Ausbildung der Hautdurchleitung in einem Längsschnitt. Der Kanal 1 ermöglicht mit seinem Lumen 2 die Durchleitung von Flüssigkeiten oder von Leitungen ins Innere des Körpers 3. An die Dreiphasenlinie 4 wird durch das Herauswachsen der Infektionsschutzmanschette 5 nach aussen, durch den Pfeil 6 gekennzeichnet, neues und steriles Material herangeführt. Dieses härtet in der Extrusionsdüse 7 aus und tritt aus ihr in der Form einer Infektionsschutzmanschette 5 ständig oder intermittierend aus. Das flüssige Material für die Manschette 5 wird durch die Rohrleitung 8 von aussen zur Extrusionsdüse 7 gefördert. Dies wird mit einer Dosierpumpe 9 bewirkt, die aus einem Vorratsbehälter 10 gespeist wird. Dieser Vorratsbehälter 10 kann nach Bedarf durch einen Port neu aufgefüllt werden. Die Dosierpumpe 9 kann eine elektrische Mikropumpe sein, eine osmotische Pumpe sein, die durch eine Batterie betrieben wird, oder aber auch eine Pumpe sein, die von Hand in zeitlichen Abständen von Tagen, Wochen oder Monaten betätigt wird. Weiterhin ist eine mechanische Pumpe mit einem Federwerk für diese Aufgabe geeignet. Die Dosierpumpe 9 kann aber auch durch ein Druckreservoir ersetzt werden, das unter einem Gas- oder Dampfdruck das Material fördert. Im Körperinnern sollen die Zellen sich mit der neu erzeugten Infektionsschutzmanschette verbinden und auf ihrer äusseren Fläche anwachsen. Es ist bakannt, dass Zellen beispielsweise auf einer Silikonkautschukoberfläche gut anwachsen. Dies wird noch durch eine rauhe Oberfläche verbessert. Deshalb kann die Extrusionsdüse 7 zur Erzeugung der Infektionsschutzmanschette mit Längsrillen versehen sein. Bei der Extrusion bildet sich so die Infektionsschutzmanschette mit vergrösserter äusserer Oberfläche und ist damit besser geeignet für eine Anhaftung der Zellen.

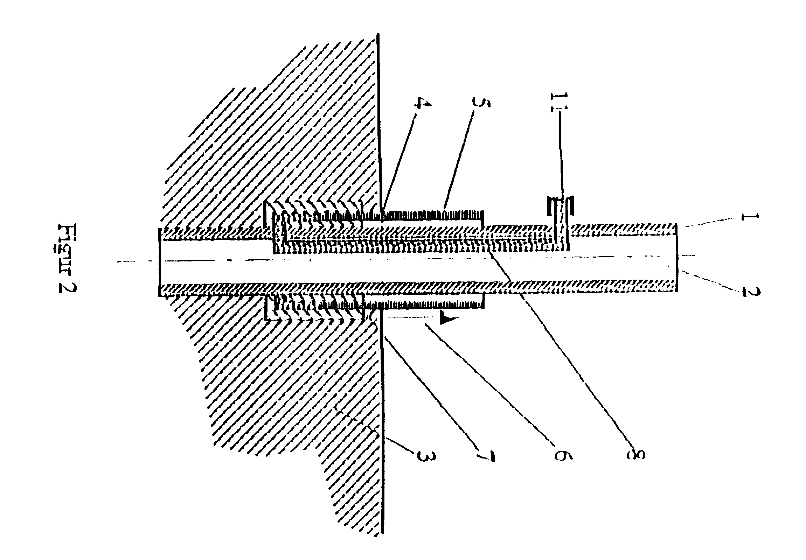

Figur 2 zeigt eine weitere Ausbildung der Hautdurchleitung in einem Längsschnitt. In diesem Fall fehlen der Vorratsbehälter 10 und die Dosierpumpe 9. Das Material für die zu bildende Infektionsschutzmanschette 5 wird durch die Rohrleitung 8 von aussen zur Extrusionsdüse 7 gefördert, indem das Material von aussen durch Druck hier in zeitlichen Abständen hineingepresst wird. Die Aushärtung erfolgt in einer von aussen nach innen vordringenden Front. Damit es bei zu langen Zeitabständen der Materialzuführung nicht zu einer Verstopfung kommt, ist der Extrusionskanal so ausgebildet, das sich der Querschnitt von innen nach aussen nicht verengt. Die Rohrleitung 8 wird in den Zwischenzeiten mit der Kappe 11 verschlossen und verhindert damit das Eindringen von Luftfeuchtigkeit ins Ausgangsmaterial und damit eine unerwünschte vorzeitige Aushärtung.

## Patentansprüche

1. Verfahren zur Bildung einer Infektionsschutzmanschette für die Hautdurchleitung eines Kanals durch die menschliche oder tierische Haut, **dadurch gekennzeichnet, dass** mittels von aussen ins Körperinnere zugeführtem körperverträglichem Kunststoff unter der Haut eine sich ständig oder in definierten zeitlichen Abständen aus dem Körper herausbewegende sterile Infektionsschutzmanschette gebildet wird, die zwischen dem Kanal und dem umgebenden Körpergewebe eine geschlossene Trennschicht bildet.

2. Verfahren nach Anspruch 1, **dadurch gekennzeichnet, dass** die Infektionsschutzmanschette aus einem flüssigen oder plastischen Material gebildet wird, das unter dem Einfluss von Wasserdampf aus dem umgebenden Körpergewebe zu einem elastomeren Material aushärtet.

3. Verfahren nach Anspruch 1 oder 2, **dadurch gekennzeichnet, dass** die Infektionsschutzmanschette im Körperinneren unmittelbar unter der Haut ihren Ursprung hat und dort fortlaufend oder intermittierend neu gebildet wird.

4. Verfahren nach einem der voranstehenden Ansprueche, **dadurch gekennzeichnet, dass** die Infektionsschutzmanschette in ihrer ausgehärteten Form von körpereigenen Zellen bewachsbar ist.

5. Verfahren nach einem der voranstehenden Ansprüche, **dadurch gekennzeichnet, dass** das Material der Infektionsschutzmanschette zusätzlich biologisch wirksame Stoffe enthält.

6. Vorrichtung zur Durchführung des Verfahrens nach einem der vorstehenden Ansprüche, **dadurch gekennzeichnet, dass** im körperäusseren Bereich eine Dosierpumpe (9) mit einem Vorratsbehälter (10) über eine im Kanal integrierte Rohrleitung (8) mit einer im Körperinneren unmittelbar unter der Haut angeordneten Extrusionsdüse strömungsmässig verbunden ist.

7. Vorrichtung nach Anspruch 6, **dadurch gekennzeichnet, dass** die Extrusionsdüse (7) eine kreisringförmige Extrusionsöffnung aufweist, die den funktionstragenden Kanal eng umschliesst.

8. Vorrichtung nach einem der voranstehenden Ansprüche, **dadurch gekennzeichnet, dass** die Extrusionsdüse zur Erzeugung der Infektionsschutzmanschette mit einer rauhen Struktur versehen ist, die ihr eine vergrösserte Oberfläche verleiht.

9. Vorrichtung nach einem der voranstehenden Ansprüche, **dadurch gekennzeichnet, dass** der Kanal für die Einleitung oder Herausleitung von Flüssigkeiten und/oder Gasen ausgebildet ist.

10. Vorrichtung nach einem der voranstehenden Ansprüche, **dadurch gekennzeichnet, dass** der gesamte Bereich, in dem das Material zugeführt wird, aus einem Werkstoff besteht, der ein Anhaften oder Ankleben des Materials verhindert.

11. Vorrichtung nach einem der voranstehenden Ansprüche, **dadurch gekennzeichnet, dass** der von aussen ins Körperinnere zugeführte körperverträgliche Kunststoff in zeitlichen Abständen durch einen nicht ständig mit dem Patienten verbundenen Zuführungsgerät erfolgt.

12. Vorrichtung nach einem der voranstehenden Ansprüche, **dadurch gekennzeichnet, dass** der von aussen ins Körperinnere zugeführte körperverträgliche Kunststoff bereits mit einem Katalysator vermischt ist und im Körper zum Elastomer aushärtet.
